# EUROPÄISCHE PATENTSCHRIFT

(11) **EP 0 797 609 B1**
(45) Veröffentlichungstag und Bekanntmachung des Hinweises auf die Patenterteilung: **10.05.2000**
(21) Anmeldenummer: 95911254.1
(22) Anmeldetag: 23.02.1995
(51) Int. Cl.: C08G 63/664, C08G 63/00, A61K 9/16, C08B 37/00

(54) **POLYESTER**
POLYESTERS
POLYESTERS

(30) Priorität: 25.02.1994 DE 4406172
(43) Veröffentlichungstag der Anmeldung: 01.10.1997
(73) Patentinhaber: SCHWARZ PHARMA AG, 40789 Monheim (DE)
(72) Erfinder: KISSEL, Thomas, D-79219 Staufen (DE); LI, Youxin, D-35039 Marburg (DE)
(86) Internationale Anmeldenummer: EP9500655
(87) Internationale Veröffentlichungsnummer: WO9523175

(56) Entgegenhaltungen:
- WO-A-93/22362
- DE-A- 3 430 852

## Beschreibung

Die Erfindung betrifft neue Polyester, bestehend aus einem Substituenten mit Elektrolyteigenschaften enthaltendem Polyol mit polymeren Hydroxycarbonsäureester, deren Herstellung sowie deren Verwendung.

Insbesondere betrifft sie neue verzweigt-kettige Ester aus Substituenten mit Elektrolyteigenschaften enthaltendem Polyol mit Polymilch- oder Copolymilchglykol-säure bestehenden Resten, deren Herstellung sowie deren Verwendung als Matrixmaterial für pharmakologisch aktive Stoffe enthaltende Depotformen.

Depotformen können auf unterschiedliche Weise wie z.B. oral, parenteral, okular, pulmonal oder durch Einstreuen in Wunden verabreicht werden. Von besonderem Interesse sind zur parenteralen Applikation geeignete Depotformen, die auch als parenterale Retardformen bezeichnet werden.

Parenterale Retardformen können als Mikropartikel, Implantate und Fasern formuliert werden. Mikropartikel sind dabei von besonderem Interesse, da sie aufgrund ihrer geringen Abmessungen in einem geeigneten Medium suspendiert, mittels einer Spritze über eine Injektionsnadel mit geringem Durchmesser vergleichsweise schmerzarm appliziert werden können.

Derartige Formulierungen sind für alle pharmakologisch aktiven Stoffe von Interesse, wenn lang anhaltende, gleichbleibende systemische oder lokale Wirkstoffkonzentrationen erwünscht sind. Besonders vorteilhaft sind sie für Wirkstoffe, die bei oraler Gabe zerstört oder unzureichend resorbiert werden und nur parenteral appliziert werden können. Letzteres ist beispielsweise bei pharmakologisch aktiven Peptiden wie z.B. Peptidhormonen oder Proteinen der Fall.

Von besonderem Interesse sind dabei Interleukine (IL-1 bis IL-15), Interferone (IFN), Neurotrophine (NT-1 bis NT-3), Colonie stimulierende Faktoren (CSF), epidermale Wachstumsfaktoren (EGF), neuronale Wachstumsfaktoren, Prolactin, Luteinisierendes Hormon-Releasing Hormon (LH-RH), Insulin, Somatostatin, Glucagon, Gastrin, Pentagastrin, Urogastron, Calcitonin, Sekretin, Enkephaline, Endorphine, Angiotensine, Renin, Bradykinin, Tyrocidin, Gramicidine, Erythropoetin (EPO), Angiopeptin, Hirudin, Oxytocin, Vasopressin, Calcitonin gene related peptide (CGRP), Brain derived growth factors (BDGF), deren synthetischen Analoga und Modifikationen, sowie deren pharmakologisch aktive Fragmente.

Im allgemeinen wird für parenterale Retardformulierungen eine möglichst konstante Wirkstofffreisetzung über den gesamten Freisetzungszeitraum angestrebt. Die Freisetzung von Wirkstoffen aus Depotformen aus bioabbaubaren Matrixpolymeren wird durch ihre Diffusionsgeschwindigkeit im Polymer und dessen Abbaugeschwindigkeit bestimmt. Zur Vermeidung von Kumulation des Matrixpolymers bei sukzessiver Applikation sollte dieses nach Beendigung der Wirkstofffreisetzung möglichst vollständig abgebaut sein.

Bioabbaubare Matrixpolymere für Wirkstoffeinbettungen wurden bereits 1973 mit US 3,773,919 beschrieben. Dabei wurden Polymere aus Hydroxycarbonsäuren, insbesondere aus Milch- und/oder Glykolsäure vorgeschlagen. Polymere aus Milch- und/oder Glykolsäure werden im Körper zu Milch- und/oder Glykolsäure hydrolysiert, welche weiter zu CO₂ und Wasser metabolisiert werden und sind damit insbesondere zur Herstellung parenteraler Retardformen geeignet.

Depotformen aus Polymilchsäure (PLA) oder Polymilchglykolsäure (PLGA), insbesondere Mikropartikel, zeigen zumeist einen mehrphasigen Freisetzungsverlauf und weisen anfänglich eine stark erhöhte Freisetzung durch an der Oberfläche befindlichen Wirkstoff auf. Insbesondere bei Peptidwirkstoffen kommt es anschließend zu einer Phase stark verminderter oder nicht vorhandener Freisetzung, die wiederum von einer späteren, durch Polymermassenabbau getragenen Wirkstoffliberation gefolgt wird. Zum Zeitpunkt der Beendigung der Wirkstofffreisetzung sind noch Polymerreste vorhanden.

EP 0 058 481 B1 beschreibt die Verwendung eines Gemisches aus PLGA, die verschiedene Molekulargewichte aufweisen. Hierdurch soll die Freisetzung linearisiert und die Abbaugeschwindigkeit dem Freisetzungszeitraum angepaßt werden. Verwendung derartiger Polymergemische stellen jedoch hohe Anforderungen an die Hydrolysestabilität des Wirkstoffes und sind nicht zur Herstellung von Mikropartikeln geeignet.

EP 0 407 617 beschreibt ein biokompatibles Polyester mit erhöhter Hydrolysegeschwindigkeit, bestehend aus an PLA oder PLGA gebundenen Sacchariden. Die Polyester werden als Matrixmaterial für Depotformen vorgeschlagen, ein entsprechendes Ausführungsbeispiel ist jedoch nicht enthalten.

Sofern mit diesen Polymeren Depotformen herstellbar sind, erscheint es zwar möglich, schneller abbaubare Depotformen zu entwickeln, das Problem der uneinheitlichen Wirkstofffreisetzung und insbesondere das Problem des initialen Burst-Effektes bleibt jedoch weiterhin bestehen.

DE 34 30 852 A1 beschreibt Ester aus Polyolen und Poly- oder Copoly-milchsäure, die ebenfalls als Matrixpolymere für Depotformen vorgeschlagen werden. Beispiel 26 enthält beispielsmäßig die in-vitro-Freisetzung von mittels Sprühtrocknung hergestellten Bromocriptin-mesylat enthaltenden, gewaschenen Mikropartikeln. Trotz Abwaschen von an der Mikropartikeloberfläche anhaftendem Wirkstoff wurden nach 24 Stunden bereits 62 % der Wirkstoffbeladung freigesetzt.

WO 93/22362 A1 offenbart Polymere aus Polyvinylalkohol mit Citronensäure oder Citronensäure/Weinsäuregemischen, welche als Zusatz zu Wasch- und Reinigungsmitteln verwendet werden.

Mit den bekannten Matrixpolymeren können oft keine Retardformen mit genügend hoher Wirkstoffbeladung hergestellt werden. Eine hohe Wirkstoffbeladung der Retardformen ist jedoch erwünscht, da die jeweils applizierbare Menge durch ihre intramuskuläre oder subkutane Verabreichung limitiert wird, und die für Retardformen angestrebten, langen Applikationsintervalle häufig nur auf diese Weise erreicht werden können.

Erhöhung der Wirkstoffbeladung führt jedoch in Abhängigkeit von den physikochemischen Eigenschaften des Wirkstoffes zumeist zu unzureichender Retardierung. Insbesondere hydrophile Wirkstoffe, beispielsweise solche, die unter physiologischen Bedingungen in dissoziierter Form vorliegen, können daher nicht in der erforderlichen, hohen Konzentration in die bekannten Matrixpolymere verbracht werden und zeigen uneinheitliche Wirkstofffreisetzung, insbesondere eine erhöhte initiale Wirkstofffreisetzung (Burst-Effekt).

Es war daher Aufgabe der vorliegenden Erfindung, Matrixpolymere zur Verfügung zu stellen, die die beschriebenen Probleme nicht aufweisen. Die Matrixpolymere sollten eine hohe Wirkstoffbeladung ermöglichen und die Wirkstoffe retardiert und ohne Schwankungen freisetzen. Dabei sollte die Geschwindigkeit und Gesamtdauer des Polymerabbaus der Geschwindigkeit und Dauer der Wirkstofffreisetzung angepaßt sein, so daß nach Freisetzungsende eine weitere Dosis der Depotform appliziert werden kann, ohne daß die Gefahr einer Kumulation des Matrixpolymers im Körper besteht.

Die erfindungsgemäßen Matrixpolymere können leicht zu Depotformen verarbeitet werden. In den bekannten Verfahren wie z.B. solvent evaporation oder Sprühtrocknung weisen sie gute Partikelbildungseigenschaften auf und sind insbesondere zur Herstellung von Mikropartikeln geeignet.

Gegenstand der Erfindung ist ein verzweigt-kettiger Ester aus einem mindestens einen Substituenten mit Elektrolyteigenschaften enthaltenden Polyol mit aus Polyhydroxycarbonsäuren bestehenden Säureresten, wobei die Polyhydroxycarbonsäurereste aus Milchsäure, Glykolsäure, β-Hydroxypropionsäure, β-Hydroxybuttersäure, δ-Hydroxyvaleriansäure und/oder ε-Hydroxycapronsäure bestehen und der Ester eine gewichtsmittlere Molekularmasse von bis zu 500.000 aufweist.

Als Substituenten mit Elektrolyteigenschaften werden solche verstanden, die im hydrophilen Milieu zumindest teilweise in dissoziierter Form vorliegen.

Die polymeren Hydroxycarbonsäurereste können aus ein, zwei, drei oder mehr der Hydroxycarbonsäuren aufgebaut sein.

Die in den erfindungsgemäßen Polyester enthaltenen Substituenten mit Elektrolyteigenschaften können aus einer starken oder schwachen Säure oder einer starken oder schwachen Base gebildet werden und können auch in Form ihrer Salze vorliegen. Bevorzugt bestehen sie aus einer starken Säure oder einer schwachen Base und liegen in Form ihrer Salze vor.

Gegenstand der Erfindung ist daher auch ein Produkt, in dem der Elektrolyteigenschaften aufweisende Substituent aus einer Sulfogruppe, einem primären, sekundären oder tertiären Amin oder einer Carboxylgruppe gebildet wird.

Das Substituenten mit Elektrolyteigenschaften enthaltende Polyol kann aus gleichen oder verschiedenen, kettenförmig miteinander verknüpften alicylischen oder aliphatischen Einheiten bestehen und eine lineare oder zyklische Struktur aufweisen.

Derartige Polyole können beispielsweise entsprechend substituierte Poly- oder Oligomere aus Kohlenhydraten sein, wie z.B. Inulin, Dextrane, Xylane, Cyclodextrine oder aus entsprechend substituierten Polymeren aus gleichen oder verschiedenen Alkeneinheiten bestehen, wie z.B. substituierter Polyvinylalkohol oder Copolymere aus substituiertem oder unsubstituiertem Polyvinylalkohol oder teilweise acetyliertem Polyvinylalkohol mit Acrylsäure, α- oder β-Methacrylsäure, Acrylamin, α- oder β-Methacrylamin, Acrylnitril oder α- oder β-Methacrylnitril.

Bevorzugt werden Dextransulfat, Diethylaminoethyl-Dextran, Xylansulfat, Diethylaminoethyl-Xylan, Cyclodextrinsulfat, teilsulfatierter Polyvinylalkohol, Copolymere aus teilsulfatiertem Polyvinylalkohol, Polyvinylalkohol oder teiweise acetyliertem Polyvinylalkohol mit Acrylsäure, Acrylamin, Acrylnitril, α- oder β-Methacrylsäure, α- oder β-Methacrylamin oder α- oder β-Methacrylnitril sowie deren Salze. Besonders bevorzugt werden dabei ihre entsprechenden Alkalisalze, insbesondere Na-Salze bzw. ihre Halogensalze, insbesondere Chloride.

Sulfatierter Polyvinylalkohol oder dessen Copolymere können hergestellt werden, in dem das entsprechende Polyvinylacetat oder dessen Copolymere in einen H₂SO₄/SO₃ enthaltenden geeigneten Alkohol wie z.B. Ethanol mittels Alkoholyse sulfatiert und anschließend neutralisiert werden. Wird die Reaktion beispielsweise in 5 % H₂SO₄/SO₃ enthaltendem Ethanol durchgeführt und neutralisiert, werden ca. 20 % der Hydroxylgruppen sulfatiert.

Polyvinylalkohol oder teilweise acetylierten Polyvinylalkohol enthaltende Copolymere können durch saure oder alkalische Hydrolyse des entsprechenden Polyvinylacetat enthaltenden Copolymers hergestellt werden.

Bevorzugt werden Polyhydroxycarbonsäuren aus Milch- und/oder Glykolsäure. Besonders bevorzugt sind Copolymere aus Milch- und Glykolsäure, dessen Säurereste aus 25 bis 50 Mol-% Glykolsäureeinheiten bestehen.

Die Milchsäureeinheiten können dabei in optisch reiner Form (D- oder L-Milchsäure) oder als Gemisch hiervon vorliegen.

Die erfindungsgemäßen substituierten Polyolester können hergestellt werden, indem ein entsprechend substituiertes Polyol mit einer oder mehreren Hydroxycarbonsäure/n in dimerer oder Laktonform in Anwesenheit eines zur ringöffnenden Polymerisation geeigneten Katalysators wie z.B. Zinn, Zink, Zinnchlorid, Zinkchlorid, Titantetrachlorid, Aluminiumchlorid, Zinnoktoat, oder Aluminiumtriisopropyloxid umgesetzt wird. Vorzugsweise finden Zinnoktoat und Aluminiumtriisopropyloxid Verwendung.

Zur Polymerisation werden die Reaktionskomponenten miteinander und mit dem Katalysator vermischt und bei erhöhter Temperatur umgesetzt.

Die entstandenen erfindungsgemäßen Verbindungen können in an sich bekannter Weise isoliert und gereinigt werden. Sie sind besonders geeignet als Depotmatrixmaterial für Arzneistoffe.

Die Ausführungsbeispiele, ohne darauf beschränkt zu sein, erläutern die Erfindung.

### Herstellung von verzweigtkettigen PLGA mit Dextransulfat-Na (DSS) als Rückgratmaterial

### Beispiel 1:

28 g D,L-Laktid (LA), 22 g Glykolid (GA) und 0,5 g DSS (Sigma, Mw 500.000) mit 1-2 SO₃-Gruppen pro Monomer wurden in einem 100 ml Stickstoffkolben vorgelegt, mit Stickstoff gespült und unter Stickstoffatmosphäre im Ölbad auf 170°C temperierten Ölbad erhitzt, bis die Monomere geschmolzen sind. Anschließend wurde unter ständigem Rühren 200 mg Zinnoktoat in die Schmelze gespritzt und die Reaktionstemperatur nach einer halben Stunde auf 150°C abgesenkt, wo die Reaktion für weitere 3,5 Stunden fortgeführt wurde. Nach Abkühlen auf Raumtemperatur wurde das Produkt in 100 ml Methylenchlorid gelöst und 3 mal mit destilliertem Wasser gewaschen, um DSS-Rückstände zu entfernen. Danach wurde die Polymerlösung durch eine Glasfilternutsche (#3) filtriert, das Produkt in Ethanol gefällt und einige Tage im Vakuum bis zur Gewichtskonstanz getrocknet.

### Herstellung von verzweigtkettigen PLGA mit Diethylaminoethyldextran (DEAED) als Rückgratmaterial

### Beispiel 2:

28 g D,L-Laktid (LA), 22 g Glykolid (GA) und 0,5 g DEAED (Sigma, Mw 500.000) mit 1-2 Aminogruppen pro Monomer wurden in einem 100 ml Stickstoffkolben vorgelegt, mit Stickstoff gespült und unter Stickstoffatmosphäre im Ölbad auf 150°C erhitzt, bis die Monomere geschmolzen sind. Anschließend wurden unter ständigem Rühren 200 mg Zinnoktoat in die Schmelze gespritzt und die Reaktion für 4 Stunden bei 150°C durchgeführt. Nach Abkühlen auf Raumtemperatur wurde das Produkt in 100 ml Methylenchlorid gelöst und 3 mal mit destilliertem Wasser gewaschen, um DEAED-Reste zu entfernen. Danach wurde die Polymerlösung durch eine Glasfilternutsche (#3) filtriert, das Produkt in Ethanol gefällt und einige Tage im Vakuum bis zur Gewichtskonstanz getrocknet.

### Herstellung von verzweigtkettigen PLGA mit einem Copolymer aus teilweise oder vollständig hydrolysiertem Polyvinylacetat und Crotonsäure (β-Methacrylsäure) als Rückgratpolymer (PVAcCA)

### 1. Hydrolyse von Poly(vinylacetat-cocrotonsäure)(PVAcCA)

### a) basische Hydrolyse

2 g PVAcCA wurden in 100 ml Methanol bei 40°C gelöst und mit 1 ml wässriger NaOH-Lösung (40%ig) versetzt, worauf innerhalb von 2-3 Min. Poly(vinylalkohol-cocrotonsäure) präzipitierte. Nach einer halben Stunde wurde das Produkt abfiltriert, einige Male mit Methanol gewaschen, 5 Stunden im Soxhlet-Apparat mit Methanol extrahiert und getrocknet. Es wurde ein weißes Produkt mit einem Verseifungsgrad > 99,5% erhalten.

### b) saure Hydrolyse

2,6 g PVAcCA wurden in 100 ml Methanol bei 50°C gelöst und unter Rühren 5 ml 50 %ige Schwefelsäure über 30 Min. langsam zugetropft. Nach 30 - 80 Min. wurde das Produkt abfiltriert, 5 Stunden im Soxhlet-Apparat extrahiert und getrocknet. Es wurde ein weißes Produkt erhalten, welches in Abhängigkeit von der Reaktionszeit einen Verseifungsgrad von 50 - 90 % aufwies.

### 2. Copolymerisation von PVAcCA mit PLGA

28 g D,L-Laktid (LA), 22 g Glykolid (GA) und 0,5 g PVAcCA wurden in einem 100 ml Stickstoffkolben vorgelegt, mit Stickstoff gespült und unter Stickstoffatmosphäre im Ölbad je nach Hydrolysegrad auf 150 - 200°C erhitzt, bis die Monomere geschmolzen sind. Anschließend wurden unter ständigem Rühren 200 mg Zinnoktoat in die Schmelze gespritzt und die Reaktion zunächst eine halbe Stunde bei 170°C und anschließend 3,5 Stunden bei 150°C durchgeführt. Nach Abkühlen auf Raumtemperatur wurde das Produkt in 100 ml Methylenchlorid gelöst und 3 mal mit destilliertem Wasser gewaschen, um PVAcCA-Reste zu entfernen. Danach wurde die Polymerlösung durch eine Glasfilternutsche (#3) filtriert, das Produkt in Ethanol gefällt und einige Tage im Vakuum bis zur Gewichtskonstanz getrocknet.

Die hergestellten Ester wurden in CDCl₃ gelöst und bei 25°C und unter Zusatz von Tetramethylsilan (TMS) als Referenz NMR-spektroskopisch charakterisiert.
¹H-NMR-Spektren

### DSS:

- d = 1,5 - 1,7: H-Atom der -CH₃ Gruppe der Laktyl-Einheit
- d = 3,8 - 4,7: Vier Peaks gleicher Intensität: H-Atome am Dextransulfat-Ring
Fünftes H-Atom ist durch CH₂-Peak von Glykolyl verdeckt
- d = 4,7 - 4,9: H-Atom der CH₂-Gruppe der Glykolyl-Einheit
- d = 5,3: H-Atom der -CH₂OSO₃Na, verdeckt durch -CH der Laktyl-Einheit (liegt für Dextran, also für -CH₂OH, bei d = 3,0)
- d = 5,0 - 5,4: H-Atom der -CH Gruppe der Laktyl-Einheit

### DEAED:

- d = 1,2 - 1,4: H-Atom der -CH₃ Gruppe des DEAE-Rests
- d = 3,1 - 4,0: Serie von Peaks: vier H-Atome am Dextran Ring, 10 H-Atome der -CH₂ Gruppen im DEAE-Rest bzw. am Dextran-Ring
- d = 4,9: H-Atom am C 1 im Dextran-Ring, verdeckt durch -CH₂ Peak der Glykolyl-Einheit

Rest wie oben, außer d = 5,3

Die Molekulargewichte der hergestellten Ester wurden mittels Gelpermeationschromatographie in Methylenchlorid bestimmt. Die Bestimmung wurde mit einer temperierten Säulenkombination (Lichrogel PS mix und Lichrogel PS 40, 10 µm, Merck) bei 25°C durchgeführt unter Verwendung eines Differenzialrefrakometers (Merck-Hitachi RI-71). Für die Kalibrierung wurden Polystyrol-Standards verwendet (Merck, Mw 3,250; 5,100; 19,600; 34,500 und 87,000).

### Polymerabbau in vitro

### Herstellung der Prüfmuster

Zur Untersuchung des Abbauverhaltens der erfindungsgemäßen Polymere wurden 10 %ige (G/V) Polymerlösungen in Methylenchlorid hergestellt. Diese wurden auf mit Teflon beschichtete Platten gegossen und das Lösungsmittel zuerst 48 h bei RT, dann im Vakuum bei Raumtemperatur entfernt. Nach Trocknung bis zur Gewichtskonstanz wurden 100 - 200 µm dicke Filme erhalten, die in 20 mm x 10 mm Teile geschnitten wurden.

### Molekularmassenabbau

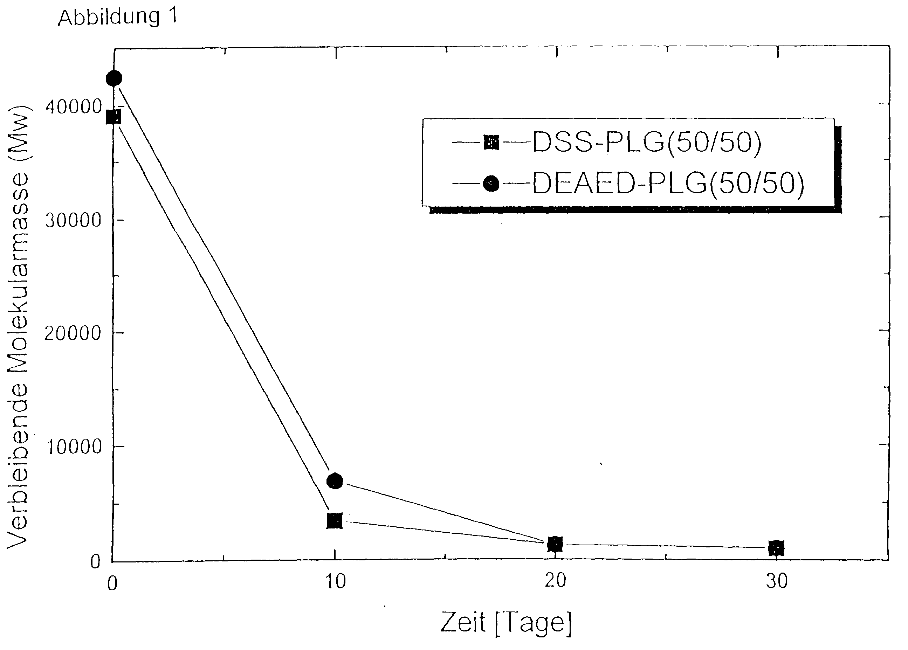

4 bis 6 der auf diese Weise hergestellten Polymerfilmteile wurden in jeweils 50 ml auf 37°C temperiertes destilliertes Wasser gegeben, nach festgelegten Zeitintervallen entnommen, im Vakuum bei Raumtemperatur bis zur Gewichtskonstanz getrocknet und mittels Gelpermeationschromatographie hinsichtlich Änderungen ihres Molekulargewichts untersucht. Abbildung 1 enthält eine Darstellung des Molekulargewichtsabbaus von aus den Polymeren 2 und 3 hergestellten Polymerfilmen.

### Polymermassenverlust

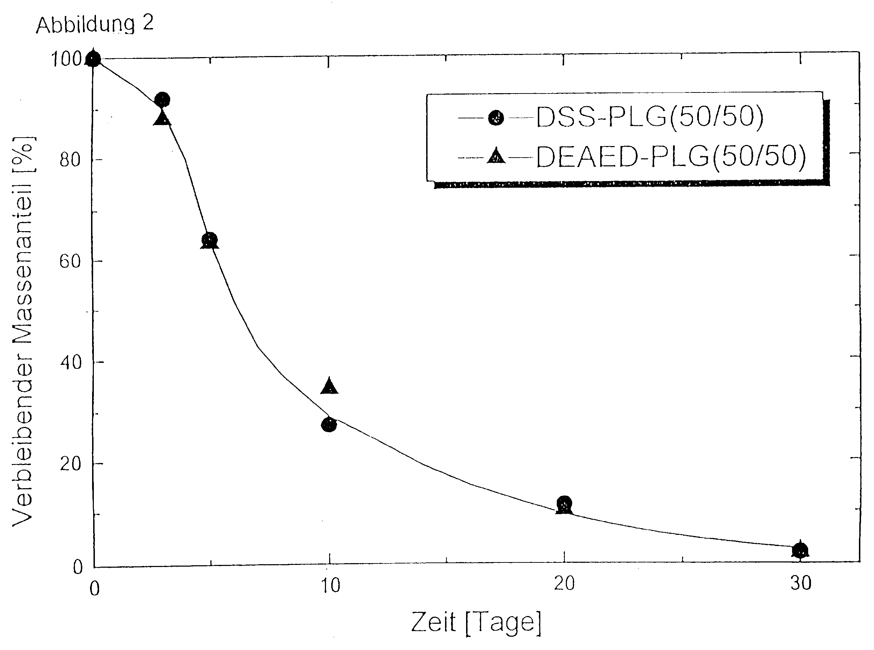

Der Gewichtsverlust wurde gravimetrisch bestimmt. Hierzu wurde ein Polymerfilmteil in ein Nylonnetz gegeben, dieses durch Erhitzen versiegelt und in ein auf 37°C temperiertes mit destilliertem Wasser befülltes Schüttelbad überführt. Nach festgelegten Zeitintervallen wurde das Netz entnommen, im Vakuum bei RT bis zur Gewichtskonstanz getrocknet und die verbleibende Masse bestimmt. Abbildung 2 enthält eine graphische Darstellung der nach den jeweiligen Zeitintervallen verbleibenden Polymermasse der aus den Polymeren 5 und 13 (Tabelle 1) hergestellen Polymerfilmen.

Die erfindungsgemäßen Polymere weisen gegenüber herkömmlichen linearen Polylaktidcoglykoliden einen deutlich erhöhten Molekularmassenabbau auf, der durch einen ebenfalls deutlich erhöhten Massenverlust des Polymers begleitet ist.

### Herstellung von mit Rinderserumalbumin (RSA) beladenen Mikropartikeln

Aus den Polymeren 5 und 13 (Tabelle 1) wurden Mikropartikel mittels solvent evaporation hergestellt. Hierzu wurde eine RSA-Lösung (25 %ig), unter intensiver Homogenisierung mit einem Zahnkranzdispergierstab, in eine 20 %ige Polymerlösung (Methylenchlorid) gegeben, so daß RSA und Polymer in einem Gewichtsverhältnis von 10 % vorlagen. Diese Emulsion wurde unter Rühren in eine 0,5 %ige PVA-Lösung eingespritzt, aus welcher die nach 3 Stunden Rühren gebildeten Mikropartikel abfiltriert wurden. Nach Trocknung (im Vacuum bei RT) wurden die Mikropartikel bei 5°C gelagert.

Der RSA-Beladungsgrad der Mikropartikel wurde nach Auflösen in Acetonitril und Extraktion mit Wasser photometrisch bei 278 nm bestimmt. Die Partikelgröße wurde mittels Laserlichtstreuung bestimmt. Die erhaltenen Ergebnisse sind in Tabelle 2 zusammengestellt.

### Wirkstofffreisetzung in vitro

Etwa 100 mg Mikropartikel (genau gewogen) wurden in verschließbare Reagenzgläser (20 ml) überführt und mit 5 ml Phosphat-Pufferlösung (pH 7,2) versetzt. Die verschlossenen Gläser wurden bei 37°C in einem rotierenden Metallblockthermostat (Rotatherm) mit 15 U/min bewegt. Nach vorbestimmten Zeitintervallen wurden die Mikropartikel bei 4000 U/min 30 min zentrifugiert, jeweils 3 ml Probenlösung entnommen, durch frische Phosphat-Pufferlösung (pH 7,2) ersetzt und die Freisetzung fortgeführt. Die RSA-Konzentration in der Probenlösung wurde photometrisch bei 278 nm bestimmt.

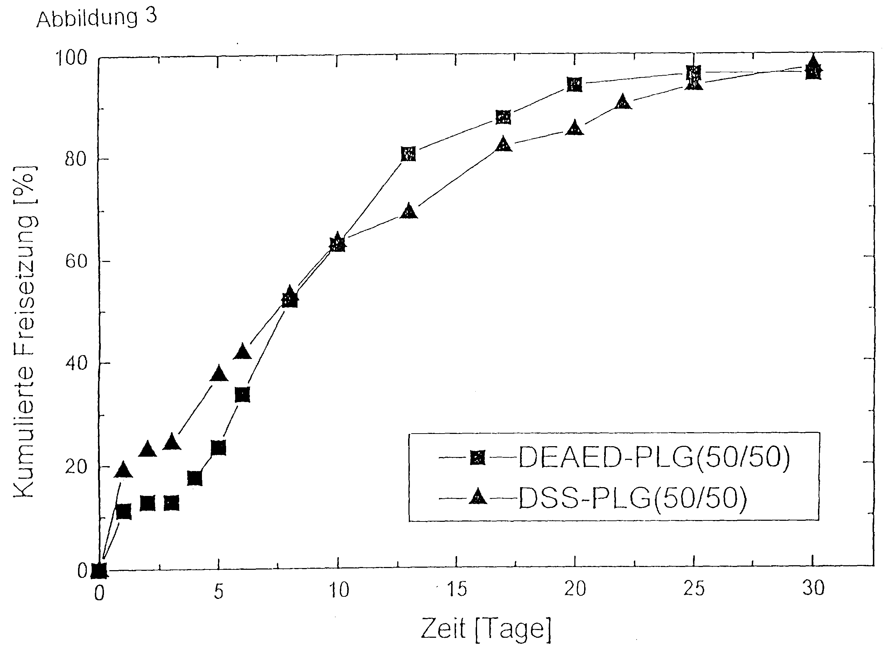

Die Ergebnisse der Freisetzungsuntersuchungen sind in Abbildung 3 dargestellt. Es ist offensichtlich, daß die Erhöhung des Anteils von Dextransulfat-Natrium im Polymer zu einer Verminderung der initial erhöhten Freisetzung führt und diese nahezu konstant über den gesamten Freisetzungszeitraum verläuft. Dies steht im deutlichen Gegensatz zum zumeist biphasischen Freisetzungsverhalten aus linearen Polylactidcoglycoliden, das durch einen hohen Bursteffekt und einer verzögert einsetzenden Wirkstoffliberation geprägt wird.

Eine Gegenüberstellung des Freisetzungsverhaltens aus den erfindungsgemäßen Polymere mit ihrem Massenverlust (vgl. Abbildung 2) zeigt weiterhin, daß ersteres weitgehend parallel zu letzterem verläuft. Das Freisetzungsverhalten erscheint somit weitgehend über den Massenabbau des Matrixpolymers kontrolliert zu werden. Die erfindungsgemäßen Polymere sind daher zur Formulierung von Depotformen besonders geeignet.

**Tabelle 1**

| Nr. | Rückgrat | Monomer LA : GA | Zinnoctoat/DSS^{a}/M (mol/mol/mol) | T(°C) | t(h) | Mw | Mw/Mn |
|---|---|---|---|---|---|---|---|
| 1 | Dex-SO₃Na | 50/50 | 2,3/14/1000 | 170 | 24 | 32.000 | 2,33 |
| 2 | Dex-SO₃Na | 50/50 | 5,3/14/1000 | 200 | 8 | 33.000 | 2,80 |
| 3 | Dex-SO₃Na | 50/50 | 1,8/14/1000 | 170 | 24 | 44.000 | 2,22 |
| 4 | Dex-SO₃Na | 50/50 | 1,2/14/1000 | 170 | 12 | 48.000 | 2,47 |
| 5 | Dex-SO₃Na | 50/50 | 1,8/14/1000 | 170 | 12 | 39.100 | 2,48 |
| 6 | Dex-SO₃Na | 50/50 | 0,42/7,2/1000 | 170 | 4 | 43.000 | 2,15 |
| 7 | Dex-SO₃Na | 50/50 | 0,85/3,0/1000 | 170 | 4 | 62.000 | 1,93 |
| 8 | Dex-SO₃Na | 50/50 | 0,85/1,5/1000 | 170 | 4 | 76.000 | 1,68 |
| 9 | Dex-SO₃Na | 50/50 | 0,85/4,4/1000 | 170 | 4 | 54.000 | 2,15 |
| 10 | Dex-SO₃Na | 50/50 | 1,8/1,5/1000 | 170 | 4 | 94.000 | 1,40 |
| 11 | Dex-SO₃Na | 75/25 | 1,3/7,5/1000 | 170 | 4 | 38.000 | 1,84 |
| 12 | Dex-SO₃Na | 1oo/0 | 1,3/7,6/1000 | 170 | 4 | 37.000 | 1,87 |
| 13 | DEAE-Dex | 50/50 | 1,8/12/1000 | 150 | 12 | 42.500 | 2,19 |
| 14 | DEAE-Dex | 50/50 | 1,8/14/1000 | 150 | 12 | 40.000 | 2,06 |
| 15 | DEAE-Dex | 75/25 | 1,8/7,8/1000 | 150 | 4 | 77.000 | 2,47 |
| 16 | DEAE-Dex | 100/0 | 1,8/7,8/1000 | 150 | 4 | 87.000 | 2,67 |

| | | | | | | | |
|---|---|---|---|---|---|---|---|
| a: oder DEAE-Dex., bezogen auf eine Ringeinheit | | | | | | | |

**Tabelle 2**

| Nr.* | LA/GA (mol) | RSA (Gew-%) | mittl. Durchmesser (µm) | Ausbeute (%) |
|---|---|---|---|---|
| 5 | 50/50 | 6,3 | 40 | 86 |
| 13 | 50/50 | 6,0 | 40 | 89 |

| | | | | |
|---|---|---|---|---|
| *) gleiche Numerierung wie Tabelle 1 | | | | |

## Patentansprüche

1. Verzweigtkettiger Ester aus einem mindestens einen Substituenten mit Elektrolyteigenschaften enthaltenden Polyol mit aus Polyhydroxycarbonsäuren bestehenden Säureresten, wobei die Polyhydroxycarbonsäurereste aus Milchsäure, Glykolsäure, β-Hydroxypropionsäure, β-Hydroxybuttersäure, δ-Hydroxyvaleriansäure und/oder ε-Hydroxycapronsäure bestehen und der Ester eine gewichtsmittlere Molekularmasse von bis zu 500.000 aufweist.

2. Produkt gemäß Anspruch 1, in dem der Elektrolyteigenschaften aufweisende Substituent aus einer Sulfogruppe, einem primären, sekundären oder tertiären Amin oder einer Carboxylgruppe gebildet wird.

3. Produkt gemäß Ansprüchen 1 oder 2, in dem das mindestens einen Substituenten mit Elektrolyteigenschaften enthaltende Polyol Dextransulfat, Diethylaminoethyl-Dextran, Xylansulfat, Diethylaminoethyl-Xylan, Cyclodextrinsulfat, teilsulfatierter Polyvinylalkohol, ein Copolymer aus teilsulfatiertem Polyvinylalkohol, Polyvinylalkohol oder teilweise acetyliertem Polyvinylalkohol mit Acrylsäure, Acrylamin, Acrylnitril, α- oder β-Methacrylsäure, α- oder β-Methacrylamin oder α- oder β-Methacrylnitril ist, sowie dessen Salze.

4. Produkt gemäß Ansprüchen 1 bis 3, in dem das mindestens einen Substituenten mit Elektrolyteigenschaften enthaltende Polyol als Alkalisalz, insbesondere Na-Salz oder als Halogensalz, insbesondere Chlorid, vorliegt.

5. Produkt gemäß Ansprüchen 1 bis 4 , in dem die aus Polyhydroxycarbonsäuren gebildeten Säurereste aus Milch- und/oder Glykolsäure bestehen.

6. Produkt gemäß Anspruch 5, dessen Säurereste aus 25 bis 50 Mol % Glykolsäureeinheiten bestehen.

7. Verwendung des Produktes gemäß Ansprüchen 1 bis 6 als Depotmatrixmaterial für Arzneistoffe.

8. Verwendung des Produktes gemäß Ansprüchen 1 bis 6 als Depotmatrixmaterial für Elektrolyteigenschaften aufweisende Arzneistoffe.

## Claims

1. Branched-chain esters from a polyol containing at least one substituent with electrolyte properties with acid groups consisting of polyhydroxycarboxylic acids having a molecular weight of up to 500,000, in which the polyhydroxy-carboxylic acids are consisting of lactic acid, glycolic acid, β-hydroxypropionic acid, β-hydroxybutyric acid, δ-hydroxy-valeric acid and/or ε-hydroxycaproic acid.

2. Product according to Claim 1, in which the substituent exhibiting electrolyte properties is formed from a sulfo group, a primary, secondary or tertiary amine or a carboxyl group.

3. Product according to Claims 1 or 2, in which the polyol containing at least one substituent with electrolyte properties is dextran sulfate, diethylaminoethyl-dextran, xylan sulfate, diethylaminoethylxylan, cyclodextrin sulfate, partially sulfonated polyvinyl alcohol, a copolymer of partially sulfonated polyvinyl alcohol, polyvinyl alcohol or partially acetylated polyvinyl alcohol with acrylic acid, acrylamine, acrylonitrile, α- or β-methacrylic acid, α- or β-methacrylamine or α- or β-methacrylonitrile, as well as their salts.

4. Product according to Claims 1 to 3, in which the polyol containing at least one substituent with electrolyte properties is present as alkali salt, especially Na salt, or as halogen salt, especially chloride.

5. Product according to Claims 1 to 4, in which the acid formed from polyhydroxycarboxylic acids consists of lactic and/or glycolic acid.

6. Product according to Claim 5, whose acid groups consist of 25 to 50 mol% glycolic acid units.

7. Use of the product according to Claims 1 to 6, as a depot matrix material for drugs.

8. Use of the product according to Claims 1 to 6, as a depot matrix material for drugs exhibiting electrolyte properties.

## Revendications

1. Ester à chaîne ramifiée composé d'un polyol contenant au moins un substituant à propriétés électrolytiques avec des radicaux acides composés d'acides polyhydroxycarboxyliques, les radicaux d'acides polyhydroxycarboxyliques étant composés de l'acide lactique, l'acide glycolique, l'acide β-hydroxypropionique, l'acide β-hydroxybutyrique, l'acide δ-hydroxyvalérianique et/ou l'acide ε-hydroxycapronique, et l'ester présentant une masse moléculaire moyenne pouvant atteindre 500 000.

2. Produit selon la Revendication 1, dans lequel le substituant à propriétés électrolytiques est formé à partir d'un groupe sulfo, d'une amine primaire, secondaire ou tertiaire ou d'un groupe carboxyle.

3. Produit selon les Revendications 1 ou 2, dans lequel le polyol contenant au moins un substituant à propriétés électrolytiques est le sulfate de dextrane, le diéthylaminoéthyl-dextrane, le sulfate de xylane, le diéthylaminoéthyl-xylane, le sulfate de cyclodextrine, l'alcool polyvinylique partiellement sulfoné, un copolymère de l'alcool polyvinylique partiellement sulfoné, de l'alcool polyvinylique ou de l'alcool polyvinylique partiellement acétylé, avec l'acide acrylique, l'acrylamine, l'acrylonitrile, l'acide α- ou β-méthacrylique, l'α- ou la β-méthacrylamine, ou l'α- ou le β-méthacrylonitrile, ainsi que leurs sels.

4. Produit selon les Revendications 1 à 3, dans lequel le polyol contenant au moins un substituant a propriétés électrolytiques est présent sous la forme d'un sel alcalin, en particulier de sel de Na, ou d'un sel halogénure, en particulier de chlorure.

5. Produit selon les Revendications 1 à 4, dans lequel les radicaux acides formés d'acides polyhydroxycarboxyliques se composent d'acide lactique et/ou glycolique.

6. Produit selon la Revendication 5, dont les radicaux acides se composent de 25 à 50 % mol. d'unités d'acide glycolique.

7. Utilisation du produit selon les Revendications 1 à 6 comme matériau de matrice "réservoir" (ou à libération contrôlée) pour des médicaments.

8. Utilisation du produit selon les Revendications 1 à 6 comme matériau de matrice "réservoir" (ou à libération contrôlée) pour des médicaments présentant des propriétés électrolytiques.
